# EUROPEAN PATENT APPLICATION

(11) **EP 4 792 746 A1**
(43) Date of publication of application: **19.08.2026**
(21) Application number: 25158038.7
(22) Date of filing: 14.02.2025
(51) Int. Cl.: A61B 6/03, A61B 90/00

(54) **INTERVENTIONAL IMAGING SYSTEM**

(71) Applicant: Koninklijke Philips N.V., 5656 AE Eindhoven (NL)
(72) Inventor: BANERJEE, Amar Satyabroto, Eindhoven (NL)
(74) Representative: Philips Intellectual Property & Standards

(57) **Abstract**

The present disclosure provides an interventional imaging system for generating medical images of an imaging area of a subject. The system comprises a smoke detection optical device configured to acquire images of the imaging area. In addition, a smoke extraction arrangement comprising one or more suction units positioned within the vicinity of the imaging area for extraction of surgical smoke is provided. A processing unit processes the acquired images, with a smoke detection algorithm or model, to detect surgical smoke in the imaging area. Then, responsive to detecting the surgical smoke in the imaging area, the processing unit controls the smoke extraction arrangement to extract the detected surgical smoke.

## Description

### FIELD OF INVENTION

The present invention relates to an interventional imaging system such as a C-arm CT machine, and more particularly to an interventional imaging system including a smoke extraction arrangement.

### BACKGROUND

Surgical procedures often generate smoke as a byproduct of tissue vaporization, cauterization, and/or laser ablation. This surgical smoke contains various harmful substances, including toxic chemicals, tissue debris, and potentially infectious particles. The presence of surgical smoke in operating rooms poses significant health risks to medical personnel and patients, including respiratory irritation, headaches, and potential long-term health effects.

Current methods for managing surgical smoke typically involve the use of manual smoke evacuation systems or localized suction devices. These systems require active operation by surgical staff and often provide incomplete coverage of the imaging area. The effectiveness of existing smoke evacuation methods is further limited by their inability to dynamically respond to the changing patterns of smoke production during surgical procedures.

The limitations of current smoke management techniques therefore expose healthcare workers to prolonged periods of smoke inhalation, potentially compromising their health and safety. Additionally, the presence of surgical smoke can obstruct the surgeon's view of the operative field, potentially impacting the precision and efficiency of surgical procedures.

Accordingly, there exists a need for an improved means for surgical smoke management.

### SUMMARY OF INVENTION

The invention is defined by the claims.

According to an aspect of the present disclosure, an interventional imaging system for generating medical images of an imaging area of a subject is provided.

The system comprises a smoke detection optical device configured to acquire images of the imaging area; a smoke extraction arrangement comprising one or more suction units positioned within the vicinity of the imaging area for extraction of surgical smoke; and a processing unit configured to: process the acquired images, with a smoke detection algorithm or model, to detect surgical smoke in the imaging area; and control the smoke extraction arrangement to extract the detected surgical smoke, responsive to detecting the surgical smoke in the imaging area.

As a result of integrating the smoke detection optical device and extraction system in the interventional imaging system (e.g., providing the smoke detection optical device and extraction system integrated with a C-arm CT machine), smoke may be detected and removed more rapidly, efficiently, and effectively than existing smoke extraction systems. This system provides real-time detection and efficient removal of surgical smoke, significantly reducing the health risks associated with exposure to harmful particles and gases in the surgical environment. The use of acquired images and advanced algorithms or models enables precise detection of smoke, allowing for targeted extraction close to the source of the surgical smoke. The automated nature of the system reduces the cognitive burden and workload of clinicians in the surgical environment, so that they may focus solely on the surgical task.

In other words, the present invention provides an innovative interventional imaging system that detects and extracts surgical smoke in real time. By leveraging imaging technology and artificial intelligence, the system offers a significant improvement over traditional manual smoke evacuation methods and room-wide automated systems, enhancing the safety and efficiency of surgical procedures.

At the core of the invention is a smoke detection optical device that acquires images of the imaging area of the interventional imaging system which may be leveraged to determine the presence of smoke, or lack thereof. More particularly, these images are processed by a sophisticated smoke detection algorithm or model, which may be implemented as a trained neural network, capable of accurately identifying the presence of surgical smoke even in complex surgical environments.

Furthermore, by incorporating a versatile smoke extraction arrangement comprising suction units strategically positioned within the vicinity of the imaging area, efficient and targeted smoke removal may be realized. By providing these suction units in close proximity to the imaging area, it may be ensured that smoke is extracted close to the source, thus reducing the chance of inhalation by individuals in and around the imaging area.

To summarize, by integrating a smoke detection and extraction system into an interventional imaging system, automated extraction of surgical smoke may be performed in an effective manner. Furthermore, by advanced smoke detection and targeted extraction capabilities, this invention addresses critical health and safety concerns in surgical environments. It offers significant advantages over existing methods, including reduced exposure to harmful surgical smoke, improved visibility for surgeons, and enhanced overall safety for both medical staff and patients.

In some embodiments, the processing unit may be configured process the acquired images, with the smoke detection algorithm or model, to detect a location of surgical smoke in the imaging area; and control the smoke extraction arrangement based on the detected location of the surgical smoke.

By not only detecting the smoke but also determining/identifying/detecting the location of smoke within the imaging area, the smoke extraction arrangement may be controlled more appropriately to extract the smoke. That is, by leveraging knowledge regarding the location of smoke, targeted extraction by the extraction arrangement may be performed by control of the arrangement. Of course, knowing the precise location of the smoke beyond just its presence in the imaging area enables better control (e.g., selective activation, control parameter choice, etc.) of the suction units.

In other words, the use of acquired images and advanced algorithms or models enables precise tracking of smoke, allowing for targeted extraction. Upon detecting smoke, the system employs the smoke detection algorithm or model, to identify the location of the smoke particles. This real-time detection and localization capability and dynamic analysis allows for immediate response to smoke generation and enables precise and targeted smoke extraction. As a result, the efficiency of the smoke removal process is improved and the exposure risks for medical personnel and patients are minimized.

In some embodiments, the processing unit may be configured to further process the acquired images, with a smoke movement analysis algorithm or model, to identify movement of the surgical smoke in the imaging area. In this case, the processing unit may be configured to detect the location of the surgical smoke further based on the identified movement.

The detection of the location of smoke may be further improved by analyzing movement of the smoke in the imaging area. Thus, the system in this case may have the ability to anticipate smoke movement patterns and control the smoke extraction system. This represents a significant advancement over typical static smoke evacuation methods, ensuring comprehensive coverage of the imaging area. Indeed, by incorporating a versatile smoke extraction arrangement comprising multiple suction units strategically positioned around the imaging area in combination with movement analysis of the smoke to further identify the location of smoke, efficient and targeted smoke removal may be realized. To this end, these units may be selectively controlled based on the identified smoke movement.

Put another way, smoke movement analysis improves appreciation of the system as to the anticipated location of the smoke relative to the suction units, which may then be more appropriately controlled with this in mind.

In some embodiments, the smoke movement analysis algorithm or model may be trained to predict a movement path of the surgical smoke in the imaging area using optical flow techniques, and the processing unit may be configured to control the smoke extraction arrangement based on the predicted movement path.

That is, optical flow techniques may be leveraged to assess at least some of the acquired images to ascertain the movement path (e.g., movement direction, change of movement direction, movement speed, etc.) of surgical smoke. Such analytical techniques may be able to accurately determine the movement path to gain a full appreciation of the flow of smoke in 3D space.

Alternatively, the smoke movement analysis algorithm or model may be a trained neural network trained to identify movement of surgical smoke in 3D space in the imaging area.

Utilizing a trained neural network for smoke movement analysis may enhance the system's accuracy and reliability in identifying movement of surgical smoke in 3D space. The neural network may be trained on labelled data comprising sequences of images of 3D spaces with smoke flowing therein. This advanced smoke movement analysis technique may enable the system to anticipate the trajectory of surgical smoke, allowing for proactive and efficient extraction before the smoke can spread throughout the imaging area.

Furthermore, the processing unit may be configured to further receive one or more operating parameters of the interventional imaging system with the smoke detection algorithm or model, and to detect the location of surgical smoke with the smoke detection algorithm or model based on the operating parameters.

By further utilizing known operating parameters (e.g., C-arm angulation) of the interventional imaging system, the identification of the location of the smoke in the imaging area may be improved. By improving the location identification, the extraction arrangement may be more effectively controlled.

The smoke extraction arrangement may comprise a plurality of suction units, and the processing unit may be configured to selectively control the suction units based on the identified movement of the surgical smoke in the imaging area and a known location of the suction units within the vicinity of the imaging area.

This selective control of multiple suction units ensures comprehensive coverage of the imaging area and improves the efficiency of smoke extraction by activating only the necessary units based on the smoke's movement. Such suction units may be strategically placed in the imaging area to ensure comprehensive coverage of smoke extraction.

In some instances, the smoke extraction arrangement may comprise a mounting unit configured to releasably attach at least one of the suction units to the interventional imaging system.

In this way, users may be able to reposition one or more of the suction units to near the source of the smoke to ensure effective extraction of the smoke. In some cases, even if the suction units are provided in the imaging area, the smoke may still travel a sizable distance before being extracted. During this traversal, the smoke may impact visibility of a user and/or be at risk of inhalation. Therefore, this helps to further alleviate this concern.

In some embodiments, the smoke detection algorithm or model may be a trained neural network trained to predict the presence of surgical smoke in acquired images. The trained neural network may be a convolutional neural network (CNN).

Utilizing a trained neural network, particularly a CNN, for smoke detection enhances the system's accuracy and reliability in identifying surgical smoke, even in complex surgical environments with varying lighting conditions and potential obstructions. The neural network may be trained on labelled data comprising images of imaging areas including and not including surgical smoke.

Furthermore, the processing unit may be configured to control at least one of a suction rate of the one or more suction units, a directionality of the one or more suction units, or a suction mode of the one or more suction units.

This fine-tuned control over suction parameters allows for adaptive smoke extraction, accommodating different surgical procedures and varying smoke production rates. Whilst requiring more complex control, this may ultimately improve efficiency and coverage of smoke extraction. Of course, additional parameters of control of the suction units may be considered.

The smoke extraction arrangement may further comprise a smoke collection chamber configured to contain surgical smoke extracted by the suction units. The smoke collection chamber may be replaceable.

The inclusion of a replaceable smoke collection chamber ensures safe containment and easy disposal of extracted surgical smoke, minimizing the risk of contamination and simplifying maintenance procedures.

The smoke extraction arrangement may further comprise a filtration system configured to purify the surgical smoke extracted by the suction units.

The addition of a filtration system further enhances the safety of the surgical environment by purifying the extracted air before release, reducing the risk of airborne contaminants.

The system may further comprise a user output interface configured to: display an alert responsive to processing unit detecting the presence of surgical smoke in the imaging area; display movement information based on the identified movement of the surgical smoke in the imaging area; and/or display parameter information describing one or more of a parameter of the smoke detection algorithm or model, a parameter of the smoke movement analysis algorithm or model, control state information of the smoke extraction arrangement by the processing unit.

This user output interface enhances the system's usability by providing real-time information and alerts to surgical staff, allowing for immediate awareness of smoke presence and system operation. Indeed, such information may enable clinicians to take proactive measures to avoid smoke inhalation, and/or provide feedback to reduce variability and peaks in smoke generation.

Additionally, the system may further comprise a user input interface configured to: receive a user input indicating a desired change to one or more of a parameter of the smoke detection algorithm or model, a parameter of the smoke movement analysis algorithm or model, and/or control of the smoke extraction arrangement by the processing unit; and generate a control signal for affecting the desired change to the processing unit.

The user input interface allows for customization and fine-tuning of the system's operation, enabling surgical staff to adapt the smoke detection and extraction process to specific surgical procedures or preferences. Indeed, different clinicians may have different preferences as to how the smoke extraction is performed.

The interventional imaging system may be a C-arm x-ray machine, an MRI machine, a CT machine, or a traditional x-ray machine.

In other words, the proposed interventional imaging system may be primarily configured for generating x-ray, CT or MRI images of a subject in the imaging area, for example. The optical device and extraction arrangement may be integrated with this system to ensure quick and efficient extraction of smoke generated during operation of the imaging system.

According to another aspect of the invention, there is provided a method for controlling an interventional imaging system comprising a smoke extraction arrangement as defined above.

The method comprises acquiring images of the imaging area; processing the acquired images, with a smoke detection algorithm or model, to detect surgical smoke in the imaging area; and controlling the smoke extraction arrangement to extract the detected surgical smoke, responsive to detecting the surgical smoke in the imaging area.

In some embodiments, the method may further comprise processing the acquired images, with the smoke detection algorithm or model, to detect a location of surgical smoke in the imaging area. In this case, the smoke extraction arrangement may be controlled based on the detected location of the surgical smoke.

These and other aspects of the invention will be apparent from and elucidated with reference to the embodiment(s) described hereinafter.

### BRIEF DESCRIPTION OF FIGURES

For a better understanding of the invention, and to show more clearly how it may be carried into effect, reference will now be made, by way of example only, to the accompanying drawings, in which:
FIG. 1 illustrates a block diagram of an interventional imaging system according to aspects of the present disclosure;
FIG. 2 illustrates a flowchart of a method for operating an interventional imaging system according to an embodiment; and
FIG. 3 is a simplified block diagram of a computer within which one or more parts of an embodiment may be employed.

### DETAILED DESCRIPTION

The invention will be described with reference to the Figures.

It should be understood that the Figures are merely schematic and are not drawn to scale. It should also be understood that the same reference numerals are used throughout the Figures to indicate the same or similar parts.

It should also be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to provide an advantage.

The present disclosure relates to concepts for an interventional imaging system that has surgical smoke detection and extraction capabilities. Surgical smoke, a byproduct of various surgical procedures, can pose significant health risks to medical personnel and patients due to the presence of hazardous chemicals, tissue debris, pathogens, and other toxic substances. The concepts described herein address the need for efficient and automated removal of surgical smoke from the operating environment of the interventional imaging system.

Specifically, imaging technology is utilized to detect the presence of surgical smoke in an imaging area. By processing acquired images (e.g., infrared images) not only can the presence of smoke be determined, but also movement of smoke within the imaging area may be assessed. This real-time detection and movement analysis capability enables effective control of a smoke extraction arrangement, in order to target extraction of the detected smoke.

The smoke extraction arrangement comprises one or more suction units strategically positioned within the vicinity of the imaging area. These suction units are designed to extract surgical smoke from the environment. Intelligent control of the smoke extraction arrangement is facilitated by smoke detection, and in some cases smoke movement analysis, algorithms and/or models (which may be AI or analysis based), ensuring targeted and efficient removal. In some cases, suction units may be removably mounted on the interventional imaging system, enabling users to reposition the suction units within the imaging area to extract smoke as desired.

By combining advanced imaging technology, sophisticated image processing algorithms or models, and automated smoke extraction, the system provides a comprehensive solution for managing surgical smoke generated during use of the interventional imaging system.

By way of brief explanation, surgical smoke is a byproduct generated during various surgical procedures, particularly those involving the use of electrosurgical devices, lasers, or ultrasonic devices. This smoke contains a complex mixture of chemical compounds, cellular debris, and potentially harmful biological materials. The production of surgical smoke is a common occurrence in operating rooms and other medical settings where tissue is cut, cauterized, or ablated.

The composition of surgical smoke varies depending on the type of procedure and the tissues involved. However, it typically contains a range of potentially hazardous substances. Exposure to surgical smoke therefore poses significant health risks to medical personnel and patients. These risks include respiratory irritation and inflammation, eye irritation, headaches and nausea, potential long-term effects on the respiratory system, and possible transmission of infectious agents.

Current methods for managing surgical smoke typically involve the use of local exhaust ventilation systems. These systems generally consist of a suction device, filters, and a collection container. While these methods provide some level of protection, they have several limitations.

Specifically, they usually require manual operation, including activation and positioning by surgical staff, which may lead to inconsistent use or reduced effectiveness due to improper placement. They may also provide limited coverage due to being limited to localized suction, which may not effectively acquire smoke produced across the entire imaging area. Furthermore, such systems often lack real-time adaptation. For example, current systems typically operate at fixed suction rates and do not adapt to varying levels of smoke production during different stages of a procedure. Finally, such systems may interfere with the surgical workflow. They may generate significant amounts of noise, even when smoke is not present in the surgical environment (which may interfere with communication in the operating room). Also, they often comprise bulky equipment or tubing associated which may impede the movement or visibility of clinicians.

In addition, room-wide smoke evacuation systems exist, but typically remove the smoke once it has already travelled a distance within the operating environment, during which time the smoke may have been inhaled. Indeed, allowing the smoke to exist in the environment may also reduce visibility for users - which may be dangerous as well as inconvenient.

The limitations of current smoke management methods when utilizing an interventional imaging system, combined with the increasing awareness of the health risks associated with surgical smoke exposure, highlight the need for more effective, automated, and comprehensive solutions that are integrated with interventional imaging systems. Improved systems for surgical smoke detection and extraction are necessary to enhance the safety of use of the interventional imaging systems and protect the health of medical personnel and patients.

The proposed interventional imaging system, including means for surgical smoke detection and extraction offers several key aspects and advantages over existing methods for managing surgical smoke in medical environments.

For each type of interventional imaging system, the optical device and extraction arrangement can be configured to optimize smoke detection and extraction without interfering with the primary imaging functions. This flexibility ensures that the benefits of real-time smoke detection and automated extraction can be realized across a wide range of surgical procedures and settings.

Moreover, disclosed embodiments provide real-time detection capabilities, enabled by advanced image processing algorithms or models. By continuously monitoring the imaging area for the presence of smoke, immediate response to changing conditions during operation of the interventional imaging system may be realized.

The automated extraction feature of the system offers several benefits:
Consistent performance: The system maintains a high level of smoke removal efficiency throughout the entire surgical procedure, reducing the risk of human error or fatigue associated with manual smoke evacuation methods.
Targeted extraction: By analyzing the movement of surgical smoke in real-time, the system can direct its extraction efforts to the areas where smoke is most concentrated, improving overall air quality in the surgical environment.
Reduced workload for medical staff. The automated nature of the system allows medical personnel to focus on the surgical procedure rather than manually managing smoke evacuation equipment.
Improved safety: By consistently and efficiently removing surgical smoke, the system helps reduce exposure to potentially harmful substances for both medical staff and patients.

These aspects and advantages collectively contribute to a more effective, efficient, and versatile solution for managing surgical smoke generated during operation of interventional imaging systems.

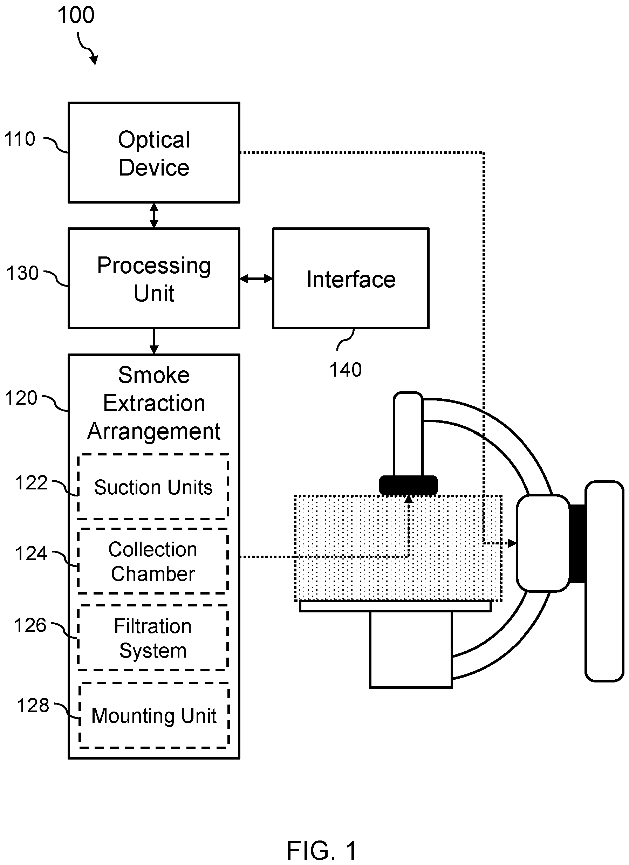

FIG. 1 illustrates a block diagram of an interventional imaging system 100 for generating medical images of an imaging area of a subject. The imaging area may be a region or 3D space configured to situate a subject of the medical imaging. For example, the imaging area may be an imaging bed. The interventional imaging system 100 comprises an innovative solution for managing surgical smoke generated in the vicinity of the interventional imaging system 100. The system 100 comprises an optical device 110, a smoke extraction arrangement 120, and a processing unit 130. The system may optionally further comprise an interface 140, which may comprise an output interface (e.g., a screen, a speaker, etc.) for providing information to a user, and/or an input interface (e.g., a touchscreen, a voice recognition system, etc.) for receiving prompts from a user.

In the case that the system 100 is a C-arm CT machine, the optical device 110 may be mounted on a sidewall of the CT scanner in view of the imaging area (indicated by the shaded area), and the smoke extraction arrangement 120 may be provided above the imaging area. Of course, embodiments are not limited hereto. For example, the optical device 110 may be mounted anywhere in view of the imaging area where the smoke may be generated and may subsequently flow, and the smoke extraction arrangement 120 may be placed anywhere where the smoke may subsequently flow.

To be clear, whilst the system 100 may be applied for smoke extraction in the vicinity of a C-arm CT scanner as illustrated, it may equally be applied in various other clinical environments, such as in the vicinity of an MRI machine, a traditional X-ray machine, etc. In each case, the optical device 110 may be provided in view of the appropriate imaging area where the smoke may be generated and where the smoke may move (e.g., in view of the patient and the area around the patient). The smoke extraction arrangement 120 may be strategically placed in each case to provide comprehensive coverage of smoke extraction.

The optical device 110 is configured to acquire images of an imaging area. The optical device 110 may continuously monitor the imaging area, providing real-time visual data for smoke detection and analysis. For example, the optical device 110 may be a high-resolution infrared camera capable of detecting thermal signatures associated with surgical smoke.

The smoke extraction arrangement 120 may comprise multiple components designed to efficiently remove surgical smoke from the area. The smoke extraction arrangement 120 includes one or more suction units strategically positioned within the vicinity of the imaging area. That is, the smoke extraction arrangement 120, at its most basic, may comprise a single suction unit. Nevertheless, the smoke extraction arrangement 120 may preferably comprise multiple suction units (e.g., four suction units each placed at a corner of the imaging area).

Each of the suction units 122 can extract surgical smoke, ensuring comprehensive coverage of the surgical field. To this end, a suction unit may ensure a high volume of air flow through the unit to extract nearby smoke. In some embodiments, each suction unit may be independently controllable. That is, the suction units may be selectively controlled such that each suction unit provides a different rate of suction (e.g., some may be turned on, whilst others are turned off). In further embodiments, various parameters of the suction units may be controlled, such as a directionality, suction rate, or a suction mode.

Furthermore, the smoke extraction arrangement 120 may further comprise one or more mounting units 128. Each mounting unit 128 may be configured to releasably attach at least one of the suction units 122 to the interventional imaging system 100. Accordingly, the suction unit 122 may be maneuverable so as to allow a user to reposition the suction unit 122 at least within the imaging area, whilst also enabling the user to refix the suction unit 122 to the system 100.

A smoke collection chamber 124 may be incorporated into the smoke extraction arrangement 120. This chamber is designed to safely contain the extracted surgical smoke and associated particles. For enhanced convenience and hygiene, the smoke collection chamber 124 may be replaceable, allowing for easy disposal and replacement between surgical procedures.

To further enhance air quality, the smoke extraction arrangement 120 may include a filtration system 126. This system is designed to purify the extracted air, removing harmful particles and chemical compounds before releasing the processed air back into the environment or a designated exhaust system. For example, advanced filtration technologies can be integrated within the suction units to further purify the captured air. These may include multi-stage filtration systems incorporating HEPA filters, activated carbon filters, and ultraviolet germicidal irradiation to effectively remove particulate matter, chemical compounds, and biological contaminants from the extracted surgical smoke.

A processing unit 130 is connected to the optical device 110 and receives the acquired images. The processing unit 130 is configured to process these images using sophisticated algorithms or models for smoke detection and movement analysis. To be clear, the processing unit 130 may be physically integrated with the optical device 110 and/or smoke extraction arrangement 120 or may be provided remotely. For example, the processing unit 130 may be provided as part of a remote device, such as a smartphone or as part of the cloud.

A smoke detection algorithm or model is employed by the processing unit 130 to detect (the presence of) surgical smoke in the acquired images. This algorithm or model is designed to identify the unique thermal signatures and patterns associated with surgical smoke, distinguishing it from other elements in the surgical environment.

To this end, the smoke detection algorithm or model may employ analytical methods or an AI model to detect smoke in the acquired images. Preferably, the smoke detection algorithm or model may be a trained neural network such as a convolutional neural network (CNN). The neural network may be trained using known training algorithms that employ a training dataset comprising a plurality of training images - some of which may include surgical smoke and some of which may not. The neural network may thus learn to differentiate between images including and not including surgical smoke. Of course, multiple different modes of detection may be employed (e.g., both analytical and AI methods) by the smoke detection algorithm or model.

Furthermore, the smoke detection algorithm or model may be used to detect/identify the location of smoke within the imaging area. That is, the acquired images may be analyzed by the smoke detection algorithm or model so as to detect where the smoke is in the imaging area. Of course, by generating this information the smoke extraction arrangement 120 may be more appropriately controlled.

In some cases, one or more operating parameters of the interventional imaging system 100 may be further provided to the smoke detection algorithm or model in order to refine the identification of the location of the smoke in the imaging area. This may improve accuracy of detection of the location of the smoke.

Upon detecting smoke, the processing unit 130 may utilize a smoke movement analysis algorithm or model to identify and predict the movement of the surgical smoke within the imaging area. That is, the smoke analysis algorithm or model may determine the movement of smoke in 3D space corresponding to the imaging area. This may be performed using a selected sequence of the images acquired by the optical device 110. For example, a selection of images immediately after the image where smoke was first detected may be analyzed.

This movement information may be used to further refine the detection of the location of smoke and therefore improve control of the smoke extraction arrangement 120.

To this end, the smoke movement analysis algorithm or model may employ analytical methods or an AI model to identify flow of smoke in the 3D area of the imaging area. Specifically, this algorithm or model may incorporate optical flow techniques to analyze the trajectory and velocity of smoke particles across sequential frames. The optical flow analysis may track the movement of smoke particles and predict their path in the 3D space.

Alternatively, the smoke movement analysis algorithm or model may be a trained neural network capable of identifying the movement of surgical smoke in three-dimensional space, providing a comprehensive understanding of smoke dynamics in the surgical environment. The neural network may be trained using known training algorithms that employ a training dataset comprising a plurality of training image sequences with corresponding known smoke flow paths. The neural network may thus learn to identify the movement of smoke in unseen images. Of course, multiple different modes of movement identification may be employed (e.g., both analytical and AI methods) by the smoke movement analysis algorithm or model.

Furthermore, enhancements to the artificial intelligence models used in smoke detection and movement analysis can be implemented through continuous training on larger and more diverse datasets. This ongoing refinement can improve the system's ability to accurately detect and predict smoke behavior across a wide range of surgical procedures and environments. Additionally, transfer learning techniques can be incorporated to adapt the models quickly to new surgical settings or types of smoke, enhancing the system's versatility.

In some embodiments, both the smoke detection algorithm or model and the smoke movement analysis algorithm or model may be implemented as part of a same process. For example, both the detection and analysis of the movement of smoke may be completed by a fully integrated AI model. Such an advanced AI model may be designed to detect and manage surgical smoke in real-time using images. The model may employ a CNN that processes high-resolution thermal images acquired during surgical procedures. It may consist of multiple convolutional layers for feature extraction, max pooling layers for down-sampling, and an attention mechanism to focus on regions of interest in the images where smoke is likely present. Complementing this, an optical flow model analyzes the detected smoke's movement direction by tracking the motion of smoke particles across sequential frames. The fully connected layers of the CNN classify the presence of smoke with high accuracy, utilizing a sigmoid activation function for binary classification. This integrated approach enables dynamic, real-time detection and directional analysis of surgical smoke, ensuring efficient and effective removal to enhance the safety of the surgical environment.

Ultimately, the processing unit 130 is configured to control the smoke extraction arrangement 120 responsive to detecting the smoke in the imaging area. In some cases, the smoke extraction arrangement 120 may be controlled based on the identified location and/or movement of the surgical smoke. This control may involve selectively activating specific suction units based on the detected smoke movement and the known locations of the units within the imaging area. The processing unit 130 may adjust various parameters of the suction units, including the suction rate, directionality, and suction mode, to optimize smoke extraction efficiency.

The interface 140 is connected to the processing unit 130, serving as both a user output interface and a user input interface. As an output interface, it can display alerts when surgical smoke is detected, provide information about smoke movement, and show various system parameters. As an input interface, it allows users to input desired changes to system settings, such as adjusting smoke detection parameters or controlling the smoke extraction arrangement 120.

For example, there may be provided customizable settings for suction power and detection sensitivity, allowing for tailored operation based on specific surgical needs. Voice and gesture controls can be added to enhance user interaction with the system 100, providing hands-free operation in sterile environments. A wireless control interface can be developed to improve mobility and ease of use for medical staff. Additionally, predictive maintenance sensors can be included to monitor the performance and condition of the system 100 components, ensuring optimal functionality and timely maintenance.

In some embodiments, the system 100 may be connected to hospital networks for centralized monitoring and data analysis, allowing for remote control and data-driven insights. By integrating the smoke detection and extraction system 100 with existing hospital information systems, medical staff can access real-time data on smoke levels and system performance from centralized monitoring stations. This network connectivity enables remote adjustment of system parameters, facilitating efficient management of multiple operating rooms or surgical suites. Additionally, the centralized data collection allows for long-term analysis of smoke production patterns across different surgical procedures, potentially leading to improved protocols for smoke management and overall operating room safety.

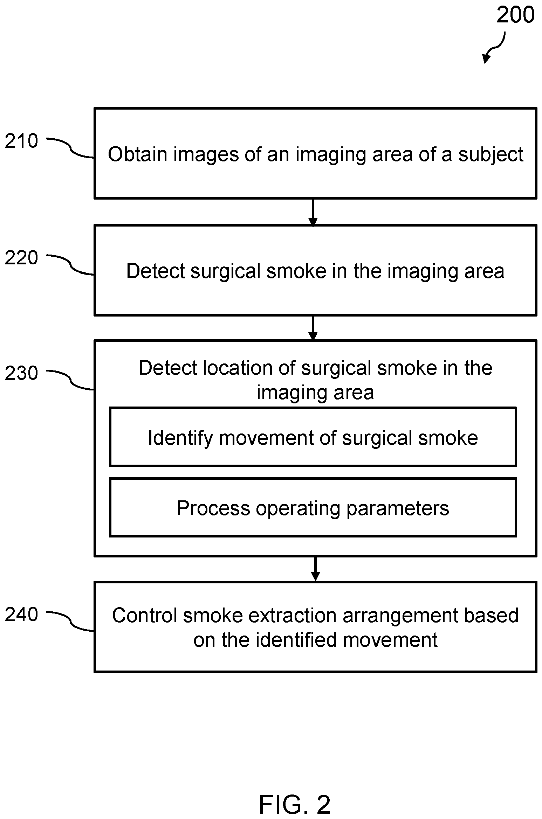

Moving on, a method of controlling an interventional imaging system for generating medical images is provided according to an embodiment is illustrated in FIG. 2, which shows a flowchart of the process. The interventional imaging system comprises means for surgical smoke detection and extraction as described in reference to FIG. 1.

In step 210, images of an imaging area of a subject are acquired. This step involves using a smoke detection optical device to acquire thermal images of the imaging area. The smoke detection optical device may be configured to continuously acquire images at a predetermined frame rate, providing real-time visual data of the imaging area. The optical device may acquire infrared images, for example.

Following the acquisition of images, the method proceeds to step 220, in which the presence of surgical smoke in the imaging area is detected using the acquired images. This detection step involves processing the images with a smoke detection algorithm or model. As described above, the smoke detection algorithm or model may be implemented as a trained neural network, such as a convolutional neural network (CNN), designed to identify the unique thermal signatures and patterns associated with surgical smoke. The algorithm or model may analyze each frame of the images (or a selection of frames), distinguishing surgical smoke from other elements in the surgical environment.

Upon detecting surgical smoke in at least one of the images, the method may advance to optional step 230, in which a location of the surgical smoke within the imaging area is identified.

This step may further involve processing the images with a smoke movement analysis algorithm or model. The smoke movement analysis algorithm or model may utilize optical flow techniques to analyze the trajectory and velocity of smoke particles across sequential frames. Alternatively, the algorithm or model may be implemented as a trained neural network capable of identifying the movement of surgical smoke in three-dimensional space. The smoke movement analysis provides a comprehensive understanding of smoke dynamics in the surgical environment, including direction and speed of smoke propagation, thereby enabling more accurate smoke location detection.

In addition, this step may further involve receiving operating parameters of the interventional imaging system, and using the operating parameters to identify the location of the smoke. In this way, more information is provided regarding the location of smoke in the imaging area, potentially improving accuracy of detection.

The final step 240 involves controlling a smoke extraction arrangement. This control may be based on the identified location and/or movement of the surgical smoke. The smoke extraction arrangement may comprise one or more suction units positioned within the vicinity of the imaging area for extraction of surgical smoke. The control of the smoke extraction arrangement may include selectively activating specific suction units based on the detected smoke movement and the known locations of the units within the imaging area. Additionally, the control may involve adjusting various parameters of the suction units, such as suction rate, directionality, or suction mode, to optimize smoke extraction efficiency.

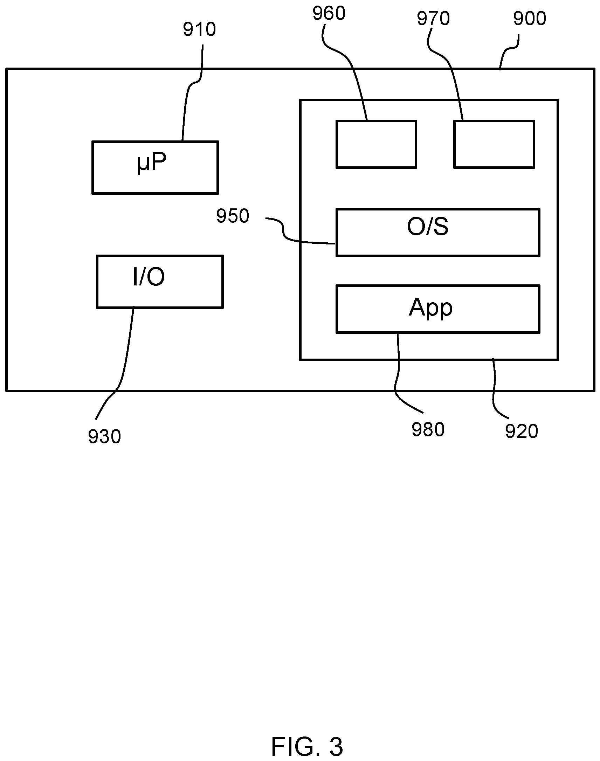

FIG. 3 illustrates an example of a computer 900 within which one or more parts of an embodiment may be employed. Various operations discussed above may utilize the capabilities of the computer. For example, one or more parts of a proposed embodiment may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example, but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor-based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc.

Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer-related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to provide an advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. An interventional imaging system for generating medical images of an imaging area of a subject, the system comprising:
a smoke detection optical device (110) configured to acquire images of the imaging area;
a smoke extraction arrangement (120) comprising one or more suction units (122) positioned within the vicinity of the imaging area for extraction of surgical smoke; and
a processing unit (130) configured to:
process the acquired images, with a smoke detection algorithm or model, to detect surgical smoke in the imaging area; and
control the smoke extraction arrangement to extract the detected surgical smoke from the imaging area, responsive to detecting the surgical smoke.

2. The system according to claim 1, wherein the processing unit (130) is configured to process the acquired images, with the smoke detection algorithm or model, to detect a location of surgical smoke in the imaging area, and to control the smoke extraction arrangement (120) based on the detected location of the surgical smoke.

3. The system according to claims 1 or 2, wherein the processing unit (130) is configured to further process the acquired images, with a smoke movement analysis algorithm or model, to identify a movement of the surgical smoke in the imaging area, wherein the processing unit is configured to detect the location of the surgical smoke further based on the identified movement.

4. The system according to claim 3, wherein the smoke movement analysis algorithm or model is trained to predict a movement path of the surgical smoke in the imaging area using optical flow techniques, and wherein the processing unit (130) is configured to control the smoke extraction arrangement based on the predicted movement path.

5. The system according to claims 3 or 4, wherein the smoke movement analysis algorithm or model is a trained neural network trained to identify movement of surgical smoke in 3D space in the imaging area.

6. The system according to any of the preceding claims 2-5, wherein the processing unit (130) configured to receive one or more operating parameters of the interventional imaging system and to detect the location of surgical smoke with the smoke detection algorithm or model based on the operating parameters.

7. The system according to any of the preceding claims 2-6, wherein the smoke extraction arrangement (120) comprises a plurality of suction units, and wherein the processing unit (130) is configured to selectively control the suction units based on the detected location of the surgical smoke and/or the identified movement of the surgical smoke in the imaging area and a known location of the suction units within the vicinity of the imaging area.

8. The system according to any of the preceding claims 1-7, wherein the smoke extraction arrangement (120) comprises a mounting unit configured to releasably attach at least one of the suction units (122) to the interventional imaging system.

9. The system according to any of the preceding claims 1-8, wherein the smoke detection algorithm or model is a trained neural network trained to predict the presence of surgical smoke in the acquired images, and optionally wherein the trained neural network is a convolutional neural network, CNN.

10. The system according to any of the preceding claims 1-9, wherein the processing unit (130) is configured to control at least one of a suction rate of the one or more suction units (122), a directionality of the one or more suction units, or a suction mode of the one or more suction units.

11. The system according to any of the preceding claims 1 to 10, further comprising a user output interface (140) configured to:
display an alert responsive to processing unit detecting the presence of surgical smoke in the imaging area;
display movement information based on the identified movement of the surgical smoke in the imaging area; and/or
display parameter information describing one or more of a parameter of the smoke detection algorithm or model, a parameter of the smoke movement analysis algorithm or model, control state information of the smoke extraction arrangement (120) by the processing unit.

12. The system according to claim 11, wherein the user input interface (140) is further configured to:
receive a user input indicating a desired change to one or more of a parameter of the smoke detection algorithm or model, a parameter of the smoke movement analysis algorithm or model, and/or control of the smoke extraction arrangement by the processing unit; and
generate a control signal for affecting the desired change to the processing unit (130).

13. The system according to any of the preceding claims 1 to 12, wherein the interventional imaging system is a C-arm x-ray machine, an MRI machine, a CT machine, or a traditional x-ray machine.

14. A method for controlling an interventional imaging system configured to generate medical images of an imaging area of a subject, the system comprising a smoke extraction arrangement (120) comprising one or more suction units (122) positioned within the vicinity of the imaging area for extraction of surgical smoke, the method comprising:
acquiring (210) images of the imaging area;
processing the acquired images, with a smoke detection algorithm or model, to detect (220) surgical smoke in the imaging area; and
controlling (240) the smoke extraction arrangement to extract the detected surgical smoke from the imaging area, responsive to detecting the surgical smoke.

15. The method of claim 14, further comprising:
processing the acquired images, with the smoke detection algorithm or model, to detect (230) a location of surgical smoke in the imaging area;
controlling the smoke extraction arrangement (120) based on the detected location of the surgical smoke.
